# FASCICULE DE BREVET EUROPEEN

(11) **EP 0 427 965 B1**
(45) Date de publication et mention de la délivrance du brevet: **08.06.1994**
(21) Numéro de dépôt: 90119710.3
(22) Date de dépôt: 15.10.1990
(51) Int. Cl.: C07C 35/08, C07C 29/145, C07C 29/20

(54) **Procédé pour la préparation de dérivés du cyclohexanol**
Verfahren zur Herstellung von Cyclohexanolderivaten
Process for the preparation of cyclohexanol derivatives

(30) Priorité: 13.11.1989 CH 4072/89
(43) Date de publication de la demande: 22.05.1991
(73) Titulaire: FIRMENICH SA, CH-1211 Genève 8 (CH)
(72) Inventeur: Weinstein, Robert M., CH-1203 Geneve (CH)
(74) Mandataire: Salvadori, Giuseppe

(56) Documents cités:
- CH-A- 416 606
- DE-A- 2 819 159
- FR-A- 2 273 783
- GB-A- 1 503 723

## Description

La présente invention a trait au domaine de la parfumerie. Elle concerne plus particulièrement un procédé nouveau pour la préparation de 4-tert-butyl-cyclohexanol éminemment sous sa forme stéréoisomérique cis. Il s'agit en l'occurrence d'un produit ayant un intérêt pour la parfumerie, spécialement en tant qu'intermédiaire pour la préparation d'esters, notamment l'ester acétique.

En particulier, l'acétate de 4-tert-butyl-cyclohexyle est un ingrédient parfumant connu depuis de nombreuses années. Mis sur le marché sous différentes dénominations, il est généralement offert sous forme d'un mélange isomérique dans lequel l'isomère cyclanique cis est présent dans des proportions variables, le plus souvent entre environ 30 et 65%. Or, il a été établi qu'il existe une différence fondamentale entre la qualité olfactive de cet isomère et celle de l'isomère trans correspondant, le premier de ces composés étant préféré [voir S. Arctander, Perfume and Flavor Chemicals, Montclair, N.J. (1969), sec.s 440 et 441]. C'est pour cette raison que de nombreux groupes de recherche se sont attelés à la préparation de cet ester sous sa forme isomérique préférentielle. A ce jour cependant, le problème de la disponibilité d'une qualité riche en dérivé cis reste posé. En effet, quoique certaines des nombreuses synthèses proposées et décrites dans l'art antérieur permettent l'obtention de mélanges isomériques de teneur adéquate en l'isomère cis, ceci est atteint au prix de solutions techniques coûteuses, ce qui rend peu rentable leur exploitation industrielle.

Le brevet US 2,927,127 du 01.03.60 décrit un procédé pour la préparation d'un mélange cis/trans de l'acétate de p-tert-butyl-cyclohexyle, dont le contenu en l'isomère cis atteint 87,5%, lequel procédé consiste en l'hydrogénation catalytique du p-tert-butyl-phénol en présence de rhodium sur un support de charbon actif et en l'acétylation du produit ainsi obtenu au moyen d'anhydride acétique. L'étape d'hydrogénation s'effectue à une pression élevée, de l'ordre de 7x10⁶ Pa.

Chemical Abstracts, vol. 80, sec. 14700s décrit également un procédé de réduction du p-tert-butyl-phénol au moyen d'une hydrogénation catalytique à l'aide de Rh sur charbon actif ou de dioxyde de ruthénium à une pression de 10x10⁶ Pa. Le contenu en l'isomère cis était de 64,1%.

Le brevet US 4,343,955 décrit un procédé pour la préparation de p-tert-butyl-cyclohexanol par une hydrogénation catalytique en présence de ruthénium sur un support d'alumine à une pression de 4x10⁶ Pa et une température de 100°C. Dans ce cas, le contenu en l'isomère as était de 74,8%.

Enfin, le brevet européen 141'054 décrit un procédé en deux étapes consistant en la réduction du p-tert-butyl-phénol à l'aide de palladium sur alumine à 120-180°C et à une pression supérieure à 20x10⁶ Pa, suivie d'une hydrogénation du produit obtenu en présence de ruthénium sur ce même support d'alumine à une température de 70-200°C et dans les mêmes conditions de pression. L'isomère cis était présent dans le mélange obtenu à raison de 46%.

Comme il a été indiqué, les procédés de l'art antérieur font appel à des méthodes impliquant des conditions drastiques de réaction, ce qui pose des problèmes techniques de nature particulière lors de leur application à l'échelle industrielle. D'autre part, il est bien connu que parmi les problèmes qui se posent dans l'application d'un procédé industriel faisant appel à des méthodes de catalyse hétérogène figurent la possibilité de recyclage du catalyseur, sa durée de vie ainsi que sa réactivation. Tous ces facteurs déterminent la valeur économique d'un procédé et sont, par conséquent, critiques lors de sa mise en application industrielle.

Nous avons maintenant découvert qu'il était désormais possible d'obtenir des mélanges de 4-tert-butyl-cyclohexanol riches en l'isomère cis par un procédé d'hydrogénation catalytique du 4-tert-butyl-phénol ou de la 4-tert-butyl-cyclohexanone en présence d'un catalyseur à base de rhodium, procédé dans lequel toutefois l'on pouvait appliquer des conditions douces de réaction et, qui plus est, présente un avantage certain vis-à-vis des méthodes connues en ce qui concerne le recyclage du catalyseur. Il est apparu en effet que lorsqu'on effectue l'hydrogénation à l'aide d'un catalyseur constitué par du rhodium préalablement déposé sur un support d'alumine en présence d'un co-catalyseur, tel le trifluorure de bore, non seulement on pouvait obtenir une excellente stéréosélectivité, mais l'on disposait également d'un système catalytique dont l'activité restait constante et suffisante pendant un nombre élevé d'opérations, ce qui rend le procédé d'autant plus économique. Quoique l'emploi de catalyseurs fluorés du type BF₃.Al₂O₃ ait été décrit dans le cas des réactions d'alkylation de systèmes aromatiques et que KF.Al₂O₃ ait été employé en tant que catalyseur dans de simples réactions d'addition [voir J. Chem. Soc 1986, 1133-39], peu de travaux font état de l'utilisation de ces catalyseurs dans des réactions d'hydrogénation et, à notre connaissance, aucune suggestion n'a jamais été émise quant à leur application dans des réactions d'hydrogénation stéréosélective de cyclohexanones ou phénols pour fournir des cyclohexanols. Le BF₃, employé comme co-catalyseur, peut se présenter en tant que tel ou sous forme d'un de ses complexes.

La présente invention a donc pour objet un procédé pour la préparation de 4-tert-butyl-cyclohexanol, éminemment sous sa forme isomérique cis, lequel procédé est caractérisé en ce qu'on soumet à hydrogénation le 4-tert-butyl-phénol ou la 4-tert-butyl-cyclohexanone en présence d'un système catalytique constitué par du rhodium, sur un support de Al₂O₃, SiO₂, TiO₂, SiO₂.Al₂O₃ ou de charbon, en combinaison avec du HBF₄ ou du BF₃.[Y]ₙ, Y désignant le groupe R₂O, R étant un groupe alkyle inférieur de C₁ à C₆, ou le groupe CH₃CO₂H, H₃PO₄ ou H₂O et l'indice n valant zéro, 1 ou 2.

La réaction qui caractérise ce procédé est illustrée par le schéma que voici : 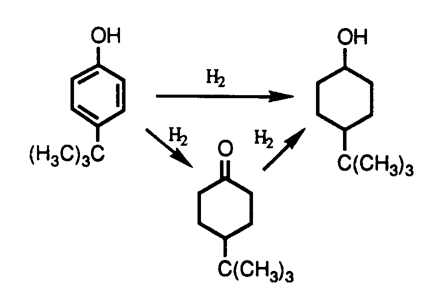
Quoique l'hydrogénation puisse s'effectuer à pression atmosphérique, nous avons remarqué que les rendements les meilleurs, ainsi que la répartition isomérique la plus favorable, étaient obtenus lorsqu'on appliquait des pressions de l'ordre de 3x10⁵ à environ 2x10⁶ Pa.

La température ne représente pas un facteur critique. Grâce à l'application du nouveau système catalytique de l'invention, il est possible de conduire la réaction à des températures voisines de la température ambiante. De bons rendements en produit final, et selon un mode préférentiel d'exécution, sont obtenus par l'emploi des températures de l'ordre de 25 à 200°C, 40°-130° étant des valeurs typiques qui peuvent s'appliquer dans la plupart des cas pratiques.

Les taux de conversion observés sont excellents et atteignent dans bien des cas 100%, tandis que la bonne stéréosélectivité du système catalytique employé se traduit par l'obtention de mélanges de 4-tert-butyl-cyclohexanol pouvant atteindre environ 90% en l'isomère préférentiel cis.

Dans les conditions de réaction définies plus haut, les temps de réaction sont de l'ordre de quelques heures. Dans bien des cas, 2 à 5 heures suffisent pour effectuer une conversion presque complète du phénol ou de la cyclohexanone de départ.

Sans vouloir préjuger de la structure réelle du système catalytique utilisé, il est apparu que l'emploi d'un "co-catalyseur" tel le BF₃ avait comme conséquence de modifier la nature de la surface du support d'alumine pour lui conférer une structure dont la stoechiométrie pouvait mieux se définir comme [Al₂O₃]_{0,8}.[AlF₃]_{0,2}. Typiquement, on a employé des catalyseurs constitués par du rhodium à 5% en poids sur de l'Al₂O₃. Il s'agit là de valeurs de concentration usuelles pour des qualités de Rh/Al₂O₃ telles que disponibles sur le marché.

L'un des paramètres critiques pour le bon déroulement de la réaction est représenté par le rapport des proportions respectives de BF₃ et du rhodium. Nous avons pu établir que ce rapport, exprimé en quantités molaires, pouvait se situer entre environ 0,5 et 15 ; toutefois, on a pu remarquer que l'augmentation de la proportion de BF₃ conduisait à la formation de produits secondaires indésirables. C'est ainsi que des proportions comprises entre 0,5 et 5 étaient parfaitement adaptées. Enfin la proportion de catalyseur, exprimée en la quantité molaire de rhodium par rapport à la quantité molaire du phénols ou de la cyclohexanone de départ, peut être de l'ordre de 0,1-0,5%.

L'hydrogénation s'effectue dans un milieu constitué par un solvant organique inerte dans les conditions de la réaction et, à cet effet, il convient d'utiliser un éther, comme par exemple le tétrahydrofuranne, ou un hydrocarbure aliphatique ou cycloaliphatique, tel par l'exemple le cyclohexane.

Le catalyseur spécifique et préférentiel employé dans le procédé de l'invention peut être préparé convenablement par imprégnation du Rh/Al₂O₃. C'est ainsi qu'un mélange de BF₃.O(C₂H₅)₂, ou trifluoroboroéthérate, et de Rh/Al₂O₃ dans de l'acétate d'éthyle, a été maintenu à environ 45°C sous atmosphère d'argon pendant 75 minutes, après quoi le produit solide a été isolé et séché. Le catalyseur ainsi obtenu est maintenu sous atmosphère de gaz inerte avant son emploi. Il s'est avéré qu'ainsi traité, le catalyseur maintenait son activité spécifique pendant au moins 7 jours. Si le système catalytique préparé comme décrit est parfaitement adapté à toute utilisation pratique selon l'invention, on peut également obtenir des systèmes efficaces par simple addition du co-catalyseur au Rh/Al₂O₃ ou Rh/C. C'est ainsi qu'on peut additionner directement de l'acide tétrafluoroborique au mélange de réaction lors de l'hydrogénation en présence de rhodium sur alumine ou sur charbon. Un tel système montre une stéréospécificité tout à fait comparable à celle du catalyseur préalablement imprégné décrit plus haut.

L'invention est illustrée d'une manière plus détaillée par les exemples suivants dans lesquels les températures sont indiquées en degrés centigrades et les abréviations ont le sens usuel dans l'art.

### Exemple 1

### Préparation du catalyseur

### Méthode générale par imprégnation

A. 10 g de rhodium à 5% (parties en poids) sur un support d'alumine commerciale équivalent à 0,50 g ou 4,86 mmole, 0,920 ml de BF₃.O(C₂H₅)₂ [distillé avant l'emploi ; 7,48 mmole] et 60 ml d'acétate d'éthyle ont été placés dans un ballon de 100 ml sous atmosphère d'azote. Après mélange, le ballon a été agité au moyen d'un évaporateur rotatif à 45° et environ 9,3x10⁴ Pa pendant 75 minutes, puis il a été concentré jusqu'à sécheresse. On a ainsi obtenu un produit solide qui a été maintenu sous atmosphère d'argon jusqu'à son utilisation.
B. 3g de rhodium à 5% (parties en poids) sur un support de TiO₂, 0,276 ml de BF₃.O(C₂H₅)₂ [distillé avant l'emploi] et 18 ml d'acétate d'éthyle ont été introduits dans un réacteur sous atmosphère d'argon. Le mélange obtenu a été maintenu sous agitation douce pendant environ 1 h 15 à 45° au moyen d'un évaporateur rotatif. On a ensuite concentré jusqu'à sécheresse. Le catalyseur ainsi obtenu a été conservé sous atmosphère d'argon jusqu'à son utilisation.

### Préparation de 4-tert-butyl-cyclohexanol

Un mélange constitué par 50 g (0,33 mole) de 4-tert-butyl-phénol, 1,35 g du catalyseur préparé comme indiqué plus haut à l'Exemple 1 A. et 100 g de cyclohexane a été introduit dans un autoclave sous atmosphère d'azote, puis de l'hydrogène a été introduit jusqu'à ce que la pression se stabilise à environ 16 bar. Le mélange de réaction a été ensuite chauffé à environ 98° et le déroulement de la réaction a été suivi par chromatographie gazeuse. Après 135 minutes, la réaction a été interrompue par refroidissement à environ 45° et l'autoclave a été purgé avec un courant d'azote. Après filtration, le catalyseur a été recueilli sous courant d'argon, lavé avec du cyclohexane et recyclé pour les opérations d'hydrogénation suivantes. Le 4-tert-butyl-cyclohexanol a été obtenu par distillation du filtrat clair avec un rendement de 98%. Le contenu isomérique était de 81,9% de l'isomère cis et 15,9% de l'isomère trans.

### Exemples 2-5

Un mélange constitué par 86,72 g de 4-tert-butyl-phénol (0,578 mole), 2,31 g de rhodium à 5% en poids sur alumine [équivalent à 1,12 mmole de rhodium], 2,50 ml d'acide tétrafluoroborique en solution à 31% [équivalent à 11,4 mmole de HBF₄] et 200 ml de tétrahydrofurane a été introduit dans un autoclave muni d'un agitateur sous atmosphère d'azote, puis de l'hydrogène a été introduit jusqu'à ce que la pression se stabilise à environ 5 bar.
Le mélange a été maintenu sous vigoureuse agitation pendant environ 24 heures à température ambiante.
Après filtration et les traitements usuels de concentration, on a obtenu le 4-tert-butyl-cyclohexanol avec un rendement presque quantitatif. Le rapport isomérique cis/trans était de 84,1/15,9.
D'autres essais ont été effectués selon la procédure générale indiquée plus haut et en remplaçant le HBF₄ par les complexes indiqués ci-après. En regard du complexe catalytique employé figure le rapport isomérique cis/trans du 4-tert-butyl-cyclohexanol obtenu.

| **Exemple** | **Catalyseur** | **Rapport isomérique cis/trans** |
|---|---|---|
| 3 | Rh/Al₂O₃ + BF₃.2CH₃CO₂H | 81/19 |
| 4 | Rh/Al₂O₃ + BF₃.H₃PO₄ | 85/13 |
| 5 | Rh/Al₂O₃ + BF₃.2H₂O | 81/18 |

### Exemple 6

### Préparation de 4-tert-butyl-cyclohexanol

Un mélange constitué par 100 g (0,649 mole) de 4-tert-butyl-cyclohexanone, 1,35 g du catalyseur préparé comme indiqué à l'Exemple 1 et 150 g de cyclohexane a été introduit dans un autoclave sous atmosphère d'azote, puis de l'hydrogène a été introduit jusqu'à ce que la pression se stabilise à environ 10⁶ Pa. Le mélange a été ensuite porté à 70° et le déroulement de la réaction a été suivi par chromatographie gazeuse.
La réaction a été interrompue par refroidissement et l'autoclave a été purgé avec un courant d'azote. Après filtration, le catalyseur a été recueilli sous courant d'argon, lavé avec du cyclohexane et recyclé pour les opérations d'hydrogénation suivantes. Le filtrat clair a été évaporé pour fournir un résidu qui, par distillation, a fourni 95,15 g d'un mélange isomérique contenant 87,7% de l'isomère cis.

### Exemple 7

### Préparation de 4-tert-butyl-cyclohexanol

Par traitement de 50 g de 4-tert-butyl-phénol en présence de 1,35 g du catalyseur préparé suivant le procédé décrit à l'Exemple 1 B. ci-dessus et en effectuant la réaction comme indiqué à l'Exemple 1, on a obtenu un mélange de 4-tert-butyl-cyclohexanol ayant un contenu en l'isomère cis de 82,9%.

### Exemple 8

### Essais de recyclage (durée de vie) du catalyseur

2 kg (13,33 mole) de 4-tert-butyl-phénol, 2 kg de cyclohexane et 28 g du catalyseur préparé comme indiqué à l'Exemple 1 A. ont été introduits dans un autoclave purgé 3 fois à l'aide d'un courant d'azote. De l'hydrogène a été ensuite introduit jusqu'à ce que la pression se stabilise à 16x10⁵ Pa. Le déroulement de la réaction a été suivi par GC et la réaction a été interrompue lorsque la conversion indique un contenu en 4-tert-butyl-cyclohexanone intermédiaire inférieur ou égal à 1%. Le mélange de réaction a été ensuite traité comme indiqué aux exemples précédents. Le catalyseur isolé par filtration a été utilisé pour des réactions successives comme indiqué au Tableau suivant.

| **Réaction n°** | **Temps de réaction (heures)** | **% Alcool-cis** | **% Alcool-trans** |
|---|---|---|---|
| 1 | 1,8 | 84,7 | 14,5 |
| 2 | 2,0 | 84,0 | 15,5 |
| 3 | 2,3 | 83,8 | 15,4 |
| 4 | 3,0 | 83,8 | 15,6 |
| 5 | 3,0 | 83,1 | 15,8 |
| 6 | 2,5 | 83,2 | 15,8 |
| 7 | 2,8 | 83,9 | 15,3 |
| 8 | 3,0 | 82,1 | 15,0 |
| | **Moyenne** | **83,6 ±,8** | **15,4 ±,4** |

## Revendications

1. Procédé pour la préparation de 4-tert-butyl-cyclohexanol éminemment sous sa forme isomérique cis, caractérisé en ce qu'on soumet à l'hydrogénation le 4-tert-butyl-phénol ou la 4-tert-butyl-cyclohexanone en présence d'un système catalytique constitué par du rhodium sur un support de Al₂O₃, SiO₂, TiO₂, SiO₂.Al₂O₃ ou de charbon, en combinaison avec du HBF₄ ou du BF₃.[Y]ₙ, Y désignant le groupe R₂O, R étant un groupe alkyle inférieur de C₁ à C₆, ou le groupe CH₃CO₂H, H₃PO₄ ou H₂O et l'indice n valant zéro, 1 ou 2.

2. Procédé selon la revendication 1, caractérisé en ce que le système catalytique est constitué par du Rh/Al₂O₃.BF₃.

3. Procédé selon la revendication 1, caractérisé en ce que l'hydrogénation s'effectue à une pression comprise entre 3x10⁵ et 2x10⁶ Pa.

4. Procédé selon la revendication 1, caractérisé en ce que l'hydrogénation s'effectue à une température comprise entre 25° et 200°C.

5. Procédé selon la revendication 4, caractérisé en ce qu'on emploie une température de 40-130°C.

6. Procédé selon la revendication 1, caractérisé en ce que le rapport des proportions respectives de BF₃ et de rhodium, exprimé en quantité molaire, est d'environ 0,5 à 15, de préférence de 0,5 à 5.

7. Procédé selon la revendication 1, caractérisé en ce que la quantité molaire de rhodium par rapport à la quantité molaire de 4-tert-butyl-phénol ou de 4-tert-butyl-cyclohexanone de départ est de 0,01 à 0,5%.

8. Procédé selon l'une des revendications précédentes, caractérisé en ce que l'hydrogénation est effectuée dans un solvant inerte dans les conditions de la réaction.

9. Procédé selon la revendication 8, caractérisé en ce que l'hydrogénation est effectuée dans le cyclohexane.

10. A titre de moyen pour la mise en oeuvre du procédé selon la revendication 1, un système catalytique constitué par du rhodium sur un support de Al₂O₃, SiO₂, TiO₂, SiO₂.Al₂O₃ ou de charbon, en combinaison avec du HBF₄ ou du BF₃.[Y]ₙ, Y pouvant désigner le groupe R₂O, R étant un groupe alkyle inférieur de C₁ à C₆, ou le groupe CH₃CO₂H, H₃PO₄ ou H₂O et l'indice n valant zéro, 1 ou 2.

## Claims

1. Process for the preparation of 4-tert-butyl-cyclohexanol, essentially in the form of its cis isomer, characterized in that 4-tert-butyl-phenol or 4-tert-butyl-cyclohexanone is subjected to hydrogenation in the presence of a catalytic system composed of rhodium on a Al₂O₃, SiO₂, TiO₂, SiO₂.Al₂O₃ or charcoal support, in combination with HBF₄ or BF₃.[Y]ₙ, wherein Y designates a R₂O group, R being a C₁ to C₆ lower alkyl radical, or the CH₃CO₂H, H₃PO₄ or H₂O group and index n is equal to zero, 1 or 2.

2. Process according to claim 1, characterized in that the catalytic system is formed of Rh/Al₂O₃.BF₃.

3. Process according to claim 1, characterized in that the hydrogenation is carried out at a pressure comprised between 3x10⁵ and 2x10⁶ Pa.

4. Process according to claim 1, characterized in that the hydrogenation is carried out at a temperature comprised between 25° and 200°C.

5. Process according to claim 4, characterized in that a temperature from 40° to 130°C is used.

6. Process according to claim 1, characterized in that the ratio between the respective proportions of BF₃ and rhodium, expressed in molar quantities, is about 0.5 to 15, preferably from 0.5 to 5.

7. Process according to claim 1, characterized in that the molar quantity of rhodium, relative to the molar quantity of starting 4-tert-butyl-phenol or 4-tert-butyl-cyclohexanone, is from 0.01 to 0.5%.

8. Process according to any one of the preceding claims, characterized in that the hydrogenation is carried out in a solvent inert under the reaction conditions.

9. Process according to claim 8, characterized in that the hydrogenation is carried out in cyclohexane.

10. As a means for carrying out the process according to claim 1, a catalytic system composed of rhodium on a Al₂O₃, SiO₂, TiO₂, SiO₂.Al₂O₃ or charcoal support, in combination with HBF₄ or BF₃.[Y]ₙ wherein Y can designate a R₂O group, R being a C₁ to C₆ lower alkyl radical, or the CH₃CO₂H, H₃PO₄ or H₂O group and index n is equal to zero, 1 or 2.

## Patentansprüche

1. Verfahren zur Herstellung von 4-t-Butylcyclohexanol, das in hohem Maße als cis-Isomeres anfällt, dadurch gekennzeichnet, daß man 4-t-Butylphenol oder 4-t-Butylcyclohexanon in Gegenwart eines Katalysatorsystems, gebildet aus Rhodium auf einem Al₂O₃-, SiO₂-, TiO₂-, SiO₂.Al₂O₃- oder Kohleträger in Kombination mit HBF₄ oder BF₃.[Y]ₙ, wobei Y die Gruppe R₂O bedeutet, worin R eine C₁-C₆-Niederalkylgruppe oder die Gruppe CH₃CO₂H, H₃PO₄ oder H₂O ist, und n 0, 1 oder 2 bedeutet, einer Hydrierung unterzieht.

2. Verfahren nach Anspruch 1, dadurch gekennzeichnet, daß das Katalysatorsystem aus Rh/Al₂O₃.BF₃ gebildet wird.

3. Verfahren nach Anspruch 1, dadurch gekennzeichnet, daß die Hydrierung bei einem Druck zwischen 3 x 10⁵ und 2 x 10⁶ Pa durchgeführt wird.

4. Verfahren nach Anspruch 1, dadurch gekennzeichnet, daß die Hydrierung bei einer Temperatur zwischen 25 und 200°C durchgeführt wird.

5. Verfahren nach Anspruch 4, dadurch gekennzeichnet, daß eine Temperatur von 40 bis 130°C angewendet wird.

6. Verfahren nach Anspruch 1, dadurch gekennzeichnet, daß das Molverhältnis von BF₃ und Rhodium etwa 0,5 bis 15, vorzugsweise 0,5 bis 5, beträgt.

7. Verfahren nach Anspruch 1, dadurch gekennzeichnet, daß die molare Menge Rhodium, bezogen auf die molare Menge des 4-t-Butylphenol oder 4-t-Butylcyclohexanon als Ausgangsmaterial, 0,01 bis 0,5 % beträgt.

8. Verfahren nach einem der vorhergehenden Ansprüche, dadurch gekennzeichnet, daß die Hydrierung in einem unter den Reaktionsbedingungen inerten Lösungsmittel durchgeführt wird.

9. Verfahren nach Anspruch 8, dadurch gekennzeichnet, daß die Hydrierung in Cyclohexan durchgeführt wird.

10. Katalysatorsystem als Mittel zur Durchführung des Verfahres nach Anspruch 1, das aus Rhodium auf einen Al₂O₃-, SiO₂-, TiO₂-, SiO₂.Al₂O₃- oder Kohleträger in Kombination mit HBF₄ oder BF₃.[Y]ₙ gebildet wird, wobei Y die Gruppe R₂O bedeuten kann, worin R eine C₁-C₆-Niederalkylgruppe oder die Gruppe CH₃CO₂H, H₃PO₄ oder H₂O ist, und n 0, 1 oder 2 bedeutet.
